# EUROPEAN PATENT APPLICATION

(11) **EP 4 331 653 A1**
(43) Date of publication of application: **06.03.2024**
(21) Application number: 22828165.5
(22) Date of filing: 31.05.2022
(51) Int. Cl.: A61M 25/00, A61M 25/06

(54) **DILATOR AND CATHETER ASSEMBLY**

(30) Priority: 21.06.2021 JP 2021102182
(71) Applicant: TERUMO Kabushiki Kaisha, Tokyo 151-0072 (JP)
(72) Inventor: OKAMURA Ryo, Ashigarakami-gun, Kanagawa 259-0151 (JP); YOSHIKAWA Shigeru, Ashigarakami-gun, Kanagawa 259-0151 (JP); MIYATA Masakazu, Wilmington, Delaware 19808 (US); ISHII Shingo, Ashigarakami-gun, Kanagawa 259-0151 (JP)
(74) Representative: Casalonga
(86) International application number: PCT/JP2022/022157
(87) International publication number: WO 2022/270247

(57) **Abstract**

Provided is a dilator and a catheter assembly capable of achieving both stable catheter insertability into a blood vessel and reduction in risk of blood vessel perforation.

A dilator (30) insertable into a catheter (20) includes a tubular shaft part (40), and a tubular flexible part (50) located on a distal end side of the shaft part (40) and formed of a resin more flexible than the shaft part (40), and includes, in the shaft part (40) and/or the flexible part (50), a physical property inclined part (42) in which rigidity gradually decreases from a proximal end side toward a distal end side.

## Description

### Technical Field

The present invention relates to a dilator to be inserted into a lumen of a catheter, and a catheter assembly in which the catheter and the dilator are connected.

### Background Art

In recent years, extracorporeal membrane oxygenation (ECMO) may be used for maintaining life for severe respiratory failure and circulatory failure that are difficult to save with a ventilator, a vasopressor, or the like. ECMO is a treatment method of removing blood from a vein, oxygenating the blood with an oxygenator, and sending the blood to an artery or vein with a pump.

In ECMO, it is necessary to indwell a catheter for removing or feeding blood in an artery or vein. When the catheter is indwelled in a blood vessel, a short sheath is inserted from the skin into the blood vessel to perform pre-dilation. Thereafter, the short sheath is removed after a guide wire is inserted into the blood vessel via the short sheath, and a catheter assembly in which the dilator is disposed inside the catheter is inserted into the blood vessel along the guide wire. Thereafter, the dilator and the guide wire are removed, and the catheter is indwelled in the blood vessel. A catheter and a dilator used for such a procedure are disclosed in, for example, Patent Literature 1.

### Citation List

### Patent Literature

Patent Literature 1: JP H08-71161 A

### Summary of Invention

### Technical Problem

A dilator to be inserted into a catheter is required to be hard to some extent in order to ensure insertability of the catheter into a blood vessel. On the other hand, when a distal end of the dilator is too hard, for example, in a case where a catheter is indwelled in a femoral vein, followability to a preceding guide wire at a tortuous blood vessel site from the femoral vein to the iliac vein and the vena cava decreases, and there is a possibility that the distal end of the dilator comes into contact with a curved inner wall surface of the vena cava and the blood vessel is perforated. Such a phenomenon easily occurs, for example, when a large-diameter catheter for COVID-19 is indwelled.

The access to a coronary artery, an abdominal blood vessel, and a lower limb blood vessel has a similar problem.

The present invention has been made to solve the above-described problems, and an object of the present invention is to provide a dilator and a catheter assembly capable of realizing both stable insertability of a catheter into a blood vessel and reduction of a risk of blood vessel perforation.

### Solution to Problem

A dilator according to the present invention that achieves the above object is a dilator insertable into a catheter, the dilator including a tubular shaft part, and a tubular flexible part located on a distal end side of the shaft part and formed of a resin more flexible than the shaft part, and including, in the shaft part and/or the flexible part, a physical property inclined part in which rigidity gradually decreases from a proximal end side toward a distal end side.

### Advantageous Effects of Invention

In the dilator configured as described above, since the proximal end side is harder than the distal end side, stable insertability into the blood vessel can be imparted to the catheter, and the risk of blood vessel perforation can be reduced since the physical property inclined part is provided. That is, the dilator can achieve both stable insertability of the catheter into the blood vessel and reduction of the risk of blood vessel perforation.
in the physical property inclined part, blending of a resin forming a part on a proximal end side with respect to the physical property inclined part and a resin forming a part on a distal end side with respect to the physical property inclined part may gradually change from the proximal end side toward the distal end side. As a result, the physical property inclined part in which the rigidity gradually decreases from the proximal end side toward the distal end side can be realized with a stable integral structure.

The flexible part may be disposed on a distal end side of a half of a total length of the dilator. As a result, since it is possible to suppress the proximal end side of the dilator from becoming too flexible, stable insertability into the blood vessel can be effectively imparted to the catheter, and the distal end side of the dilator becomes flexible, so that the risk of blood vessel perforation can be effectively reduced.

The flexible part may include, at a distal end part, a tapered part in which an outer diameter gradually decreases toward a distal end side. As a result, the distal end part of the flexible part gradually becomes softer and thinner toward the distal end side, so that the risk of blood vessel perforation can be reduced while the insertability of the flexible part is improved.

The physical property inclined part may be disposed in the shaft part. As a result, since the physical property inclined part is disposed on the proximal end side as compared with the case where the physical property inclined part is disposed in the flexible part, a wide range of a distal end region of the dilator is easily bent. Therefore, the risk of blood vessel perforation by the dilator can be reduced.

A hardness ratio may be 1.1 ≤ H_{A}/H_{B} ≤ 2.0, where H_{A} is a hardness of the resin forming the part on the proximal end side with respect to the physical property inclined part, and H_{B} is a hardness of the resin forming the part on the distal end side with respect to the physical property inclined part. As a result, the dilator can achieve both stable insertability of the catheter into the blood vessel due to the fact that the proximal end side of the shaft part is harder than the distal end side and reduction of the risk of blood vessel perforation due to the physical property inclined part in a well-balanced manner.

A catheter assembly according to the present invention for achieving the above object includes: a dilator including a tubular shaft part, and a tubular flexible part located on a distal end side of the shaft part and formed of a resin more flexible than the shaft part, and including, in the shaft part and/or the flexible part, a physical property inclined part in which rigidity gradually decreases from a proximal end side toward a distal end side; and a catheter connectable to the dilator and covering at least a part of the dilator in a state of being connected to the dilator.

The catheter assembly configured as described above can achieve both stable insertability of the catheter into the blood vessel by the shaft part having high rigidity and reduction of the risk of blood vessel perforation by the physical property inclined part.

In a state where the catheter is connected to the dilator, a distal end of the physical property inclined part may be located closer to a proximal end side than a distal end of the catheter. As a result, the physical property inclined part is disposed closer to the proximal end side than the distal end of the catheter in the catheter assembly, and the tapered part, which is a part where rigidity decreases toward the distal end side, a step part between the distal end of the catheter and an outer peripheral surface of the dilator, and the physical property inclined part are dispersedly disposed in a wide range of the distal end part of the catheter assembly. Therefore, it is possible to moderate the physical property inclination of the entire catheter assembly and suppress the instability of the insertability of the catheter into the blood vessel due to the rapid physical property inclination.

In a state where the catheter is connected to the dilator, a proximal end of the physical property inclined part may be located closer to a distal end side than a distal end of the catheter. As a result, in a case where the catheter body is thin in a radial direction and easily kinked, a wide range including the distal end of the catheter can be supported by the part having higher rigidity on the proximal end side than the physical property inclined part of the dilator. Therefore, it is easy to insert and indwell the catheter into the blood vessel without kinking the catheter.

In a state where the catheter is connected to the dilator, a distal end of the catheter may be located in a middle of the physical property inclined part in an axial direction of the dilator. As a result, in a case where a material of the catheter is less than or equal to a hardness of the flexible part of the dilator, the catheter easily bends together with the dilator, and a clearance is less likely to occur between the catheter and the dilator during skin puncture. Therefore, when the catheter assembly is pushed in a distal direction, it is possible to prevent the distal end of the catheter from being caught or turned.

The catheter may include a catheter physical property inclined part in which rigidity gradually decreases from a proximal end side toward a distal end side. As a result, the catheter assembly hardly generates a gap between the dilator and the catheter when crossing a large tortuosity, and can smoothly cross the tortuosity.

In a state where the catheter is directly or indirectly connected to the dilator, a distal end of the physical property inclined part of the dilator may be located closer to a proximal end side than a distal end of the catheter physical property inclined part, and a proximal end of the physical property inclined part of the dilator may be located closer to a proximal end side than a proximal end of the catheter physical property inclined part. As a result, the catheter assembly includes a part where rigidity gradually decreases from the proximal end side toward the distal end side close to both the catheter and the dilator. For this reason, rigidity of the catheter assembly gradually decreases from the proximal end side toward the distal end side as a whole including the catheter and the dilator. Therefore, the catheter assembly can effectively reduce the risk of blood vessel perforation while having high torque transmission.

### Brief Description of Drawings

Fig. 1 is a plan view illustrating a catheter assembly according to a first embodiment.
Fig. 2 is a plan view illustrating a state in which a catheter and a dilator of the first embodiment are connected.
Fig. 3 is a schematic diagram illustrating a state in which the catheter assembly is inserted from a femoral vein and reaches a vena cava.
Fig. 4 is a plan view illustrating a catheter assembly according to a second embodiment.
Fig. 5 is a plan view illustrating a catheter assembly according to a third embodiment.
Fig. 6 is an exploded plan view illustrating each configuration of a catheter assembly according to a fourth embodiment.
Fig. 7 is a plan view illustrating the catheter assembly according to the fourth embodiment.
Fig. 8 is a plan view illustrating a modification example of the catheter assembly according to the fourth embodiment, in which (A) illustrates a first modification example, (B) illustrates a second modification example, (C) illustrates a third modification example, and (D) illustrates a fourth modification example.
Fig. 9 is a schematic diagram illustrating a state in which the catheter assembly according to the fourth embodiment is inserted from a radial artery and reaches a lower limb artery.

### Description of Embodiments

Embodiments of the present invention will be described below with reference to the drawings. Note that dimensional ratios in the drawings may be exaggerated and different from actual ratios for convenience of description. In the following description, a side of the catheter assembly to be operated by an operator will be referred to as a "proximal end side", and a side to be inserted into a living body will be referred to as a "distal end side".

### <First Embodiment>

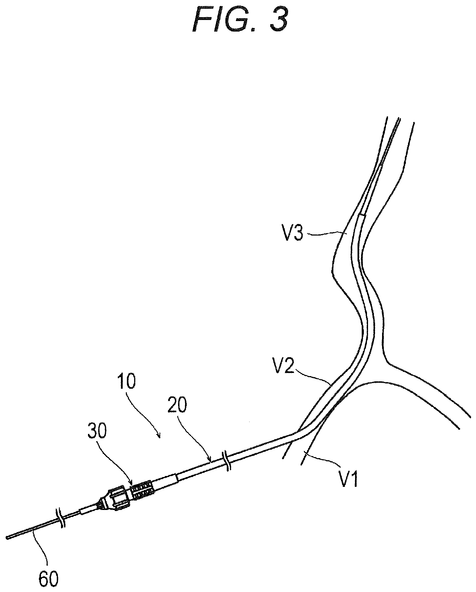

As illustrated in Fig. 3, a catheter assembly 10 according to a first embodiment of the present invention is used to insert and indwell a catheter 20 mainly for blood removal from a femoral vein V1 of a right thigh or a left thigh in a procedure (ECMO) of removing blood from a vein, oxygenating the blood with an oxygenator, and sending the blood to an artery or a vein by a pump. Note that the catheter assembly 10 may be used for ECMO blood delivery. Furthermore, a blood vessel into which the catheter 20 is inserted and indwelled is not particularly limited. Thus, the catheter 20 may be inserted, for example, from a vein other than the femoral vein V1 of the right thigh or left thigh or from a carotid artery.

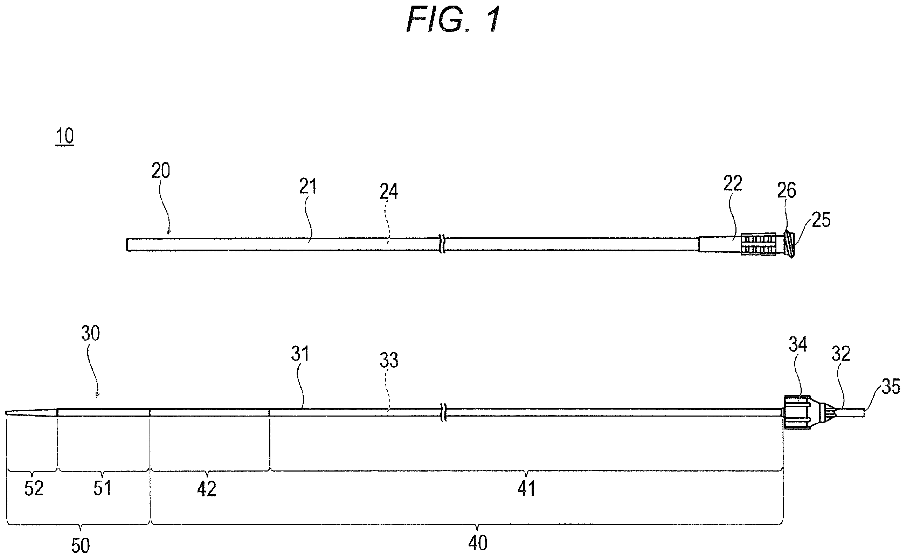

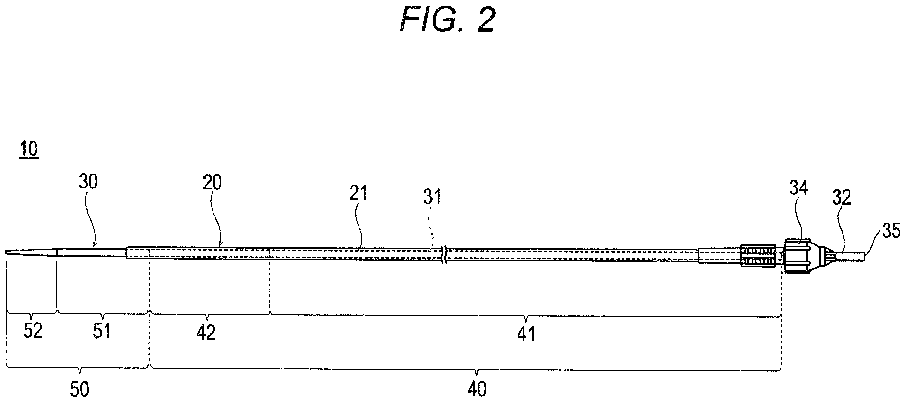

As illustrated in Figs. 1 and 2, the catheter assembly 10 according to the first embodiment includes the catheter 20 that provides a flow path of blood and a dilator 30 that functions as a core material for inserting the catheter 20 to a position for the purpose.

The catheter 20 includes a tubular catheter body 21 having flexibility and a catheter hub 22 fixed to a proximal end of the catheter body 21. The catheter 20 may also be referred to as a cannula.

The catheter body 21 is formed of a flexible tubular body, and a catheter lumen 24 is formed substantially at a center of the catheter body over the entire length of the catheter body 21. A constituent material of the catheter body 21 is not particularly limited, and examples thereof include polyester elastomer and urethane. In the catheter body 21, a coil obtained by spirally winding a wire rod or a blade obtained by braiding a plurality of wire rods into a tubular shape may be embedded as a reinforcing body. Note that the catheter body 21 may not include a reinforcing body.

The catheter hub 22 is fixed to a proximal end of the catheter body 21. The catheter hub 22 has a lumen communicating with the catheter lumen 24 and is opened at a catheter hub opening 25 on a proximal end side. A male screw 26 is formed on an outer peripheral surface of a proximal end part of the catheter hub 22. The male screw 26 can be screwed with a female screw (not illustrated) formed on an inner peripheral surface of a rotation operation unit 34 (to be described later) rotatably provided on a dilator hub 32. The male screw 26 and the female screw constitute a lock mechanism that holds a state in which the catheter hub 22 and the dilator hub 32 are connected. Note that the configuration of the lock mechanism is not particularly limited as long as the catheter hub 22 and the dilator hub 32 can be connected. For example, the lock mechanism may have a structure in which the outer peripheral surface of the dilator hub 32 having an outer diameter decreasing in a distal direction is pushed in the distal direction to be tapered and fitted to the inner peripheral surface of the catheter hub 22 having an inner diameter decreasing in the distal direction. Alternatively, the catheter hub 22 may have a straight connector in the form of a barbed fitting that can be inserted into and coupled to the lumen of the dilator hub 32.

Although the dilator 30 is inserted from the catheter hub opening 25 in an assembled state, the catheter hub 22 functions as a port for removing or feeding blood after the dilator 30 is removed.

An outer diameter of the catheter body 21 is not particularly limited, but is preferably 4.3 mm or more and 11 mm or less. An inner diameter of the catheter body 21 is not particularly limited, but is preferably 3.3 mm or more and 10 mm or less. An effective length in an axial direction of the catheter body 21 (a length in the axial direction from the distal end of the catheter hub 22 to the distal end of the catheter body 21) is not particularly limited, but is preferably 300 mm or more and 500 mm or less. In a case where the catheter 20 is inserted from the carotid artery, the effective length of the catheter body 21 in the axial direction is preferably about 300 mm.

The dilator 30 includes a dilator body 31 that can be inserted into the catheter body 21, and the dilator hub 32 fixed to a proximal end of the dilator body 31. In a central part of the dilator body 31, a dilator lumen 33 is formed along an axis extending from a distal end to a proximal end of the dilator 30. The dilator body 31 includes a tubular shaft part 40 and a flexible part 50 located on a distal end side of the shaft part 40.

The shaft part 40 preferably has a substantially constant outer diameter along an axial center, but is not limited thereto. An outer peripheral surface of the shaft part 40 is preferably circular in a cross section orthogonal to the axial center, but is not limited thereto. Since the dilator body 31 is disposed in the catheter body 21, the outer peripheral surface of the shaft part 40 is in contact with an inner peripheral surface of the catheter body with substantially no gap therebetween, or is adjacent to the inner peripheral surface of the catheter body 21 with a minute gap therebetween. For this purpose, an outer diameter of the shaft part 40 is substantially equal to an inner diameter of the catheter body 21 or slightly smaller than the inner diameter of the catheter body 21.

The shaft part 40 includes a shaft base part 41 having uniform bending rigidity along an axial center of the dilator 30, and a physical property inclined part 42 disposed on a distal end side of the shaft base part 41. The physical property inclined part 42 is formed such that the bending rigidity gradually decreases from a proximal end side toward a distal end side.

The flexible part 50 is formed in a tubular shape by a distal end side resin B that is more flexible than a proximal end side resin A forming the shaft part 40. An outer peripheral surface of the flexible part 50 is preferably circular in a cross section orthogonal to the axial center, but is not limited thereto. The flexible part 50 includes a flexible base part 51 disposed on a proximal end side and connected to the shaft part 40, and a tapered part 52 whose outer diameter gradually decreases toward a distal end side on the distal end side of the flexible base part 51. The flexible base part 51 is a part having an outer diameter substantially equal to an outer diameter of the shaft part 40. The flexible part 50 is disposed on the distal end side with respect to a position of a half of the entire length in the axial direction of the dilator 30.

In a case where a hardness of the proximal end side resin A forming the shaft base part 41 is defined as H_{A}, and a hardness of the distal end side resin B forming the flexible part 50 is defined as H_{B}, a hardness ratio (H_{A}/H_{B}) is preferably 1.1 ≤ H_{A}/H_{B} ≤ 2.0, and more preferably 1.2 ≤ H_{A}/H_{B} ≤ 1.6. When the hardness ratio (H_{A}/H_{B}) is too large, a change in rigidity in the physical property inclined part 42 becomes rapid, kink is likely to occur, and it becomes difficult to stabilize the insertability of the catheter 20 supported by the shaft part 40. Furthermore, when the hardness ratio (H_{A}/H_{B}) is too large, it is difficult to select a material, which is not realistic. When the hardness ratio (H_{A}/H_{B}) is too close to 1, a rigidity change in the physical property inclined part 42 hardly occurs, and the effect of reducing the risk of blood vessel perforation hardly occurs. When the hardness ratio (H_{A}/H_{B}) is within an appropriate range, it is possible to achieve both stable insertability of the catheter 20 due to the fact that the proximal end side of the shaft part 40 is harder than the distal end side and reduction of the risk of blood vessel perforation due to the physical property inclined part 42 in a well-balanced manner.

In the physical property inclined part 42, it is preferable that the blending of the proximal end side resin A and the distal end side resin B gradually changes from the proximal end side to the distal end side. As an example, the proximal end side resin A is high density polyethylene, and the distal end side resin B is low density polyethylene. Since the proximal end side resin A and the distal end side resin B are materials having the same composition with different hardness, they can be mixed with high compatibility. Therefore, when the shaft part 40 is molded, the material is extruded while gradually changing the blending of the proximal end side resin A and the distal end side resin B by extrusion molding, whereby the physical property inclined part 42 in which the blending of the proximal end side resin A and the distal end side resin B gradually changes can be molded. In the extrusion molding, when the proximal end side resin A and the distal end side resin B are mixed with different screws and extruded, the rotation speed of each screw is adjusted, so that the physical property inclined part 42 in which the blending of the proximal end side resin A and the distal end side resin B gradually changes can be molded. Note that the proximal end side resin A and the distal end side resin B may not be materials having the same composition as long as they can be mixed. Furthermore, the proximal end side resin A and the distal end side resin B are not limited to polyethylene as long as they are resins, and may be, for example, vinyl chloride, urethane, or the like. Furthermore, the physical property inclined part 42 may not have a structure in which the blending of the proximal end side resin A and the distal end side resin B gradually changes from the proximal end side to the distal end side. For example, the physical property inclined part 42 may be formed by radially overlapping an inner layer (or an outer layer) formed of the proximal end side resin A which gradually becomes thinner in the distal direction from the shaft base part 41 and an outer layer (or an inner layer) formed of the distal end side resin B which gradually becomes thinner in the proximal direction from the flexible part 50 so that the rigidity gradually changes.

A Shore D hardness of the proximal end side resin A is preferably D52 or more and D78 or less, more preferably D57 or more and D70 or less, and still more preferably D59 or more and D68 or less. Note that the hardness of the proximal end side resin A is not particularly limited.

A Shore D hardness of the distal end side resin B is not particularly limited as long as it is lower than the hardness of the proximal end side resin A, and is preferably D29 or more and D57 or less, more preferably D31 or more and D54 or less, and still more preferably D38 or more and D45 or less.

A length of the physical property inclined part 42 in the axial direction is not particularly limited, but is preferably 4 mm or more and 50 mm or less, more preferably 10 mm or more and 40 mm or less, and still more preferably 15 mm or more and 30 mm or less.

A position of the physical property inclined part 42 in the axial direction is not particularly limited, but is preferably 30 mm or more and 300 mm or less, and more preferably 50 mm or more and 150 mm or less from the distal end toward the proximal end of the dilator 30.

Note that the physical property inclined part 42 may be defined to be disposed at a part on the proximal end side of the flexible part 50, or may be defined to be disposed at both a part on the distal end side of the shaft part 40 and a part on the proximal end side of the flexible part 50.

An outer diameter of the shaft part 40 is not particularly limited, but is preferably 3.2 mm or more and 9.9 mm or less. Inner diameters of the shaft part 40 and the flexible part 50 are not particularly limited, but are preferably 1 mm or more and 7.9 mm or less. An overall length of the dilator 30 in the axial direction is not particularly limited, but is preferably 550 mm or more and 800 mm or less.

The dilator hub 32 is fixed to the base end of the dilator body 31. The dilator hub 32 has a lumen communicating with the dilator lumen 33 and is opened at the dilator hub opening 35 on the proximal end side. The rotatable rotation operation unit 34 is provided on an outer peripheral surface of the dilator hub 32. A female screw that can be screwed with the male screw 26 of the catheter hub 22 is formed on an inner peripheral surface of the rotation operation part 34.

In the assembled state in which the dilator 30 is connected to the catheter 20, the distal end and the proximal end of the tapered part 52 are located closer to the distal end side than the distal end of the catheter body 21 as illustrated in Fig. 2. Furthermore, in the assembled state, a part of the flexible base part 51 on the proximal end side is located closer to the proximal end side than the distal end of the catheter body 21. Furthermore, in the assembled state, the distal end of the physical property inclined part 42 is located closer to the proximal end side than the distal end of the catheter body 21. In the assembled state, the proximal end of the physical property inclined part 42 is located closer to the distal end side than the catheter hub 22. That is, in the assembled state, the physical property inclined part 42 is surrounded by the catheter body 21.

Next, a method of using the catheter assembly 10 according to the first embodiment will be described. Here, as illustrated in Fig. 3, a case where the catheter assembly 10 is inserted from the femoral vein V1 and indwelled in a vena cava V3 through a iliac vein V2 will be described as an example.

First, before the catheter assembly 10 is introduced into the blood vessel, the catheter 20 and the dilator 30 are connected to be in an assembled state. At the time of connection, the dilator 30 is inserted into the catheter hub opening 25 from a side of the dilator body 31, the rotation operation unit 34 of the dilator hub 32 is rotated, and the female screw of the rotation operation unit 34 is screwed to the male screw 26 of the catheter hub 22. As a result, the catheter 20 and the dilator 30 are connected. Therefore, the catheter 20 and the dilator 30 can be integrally operated when the catheter assembly 10 is inserted into the blood vessel.

Next, the operator punctures the femoral vein V1 by a known method, inserts a short sheath, and performs pre-dilation. Next, a guide wire 60 is inserted into the blood vessel through the short sheath, and the short sheath is removed. Subsequently, the catheter assembly 10 is pushed through the iliac vein V2 to the vena cava V3 in the assembled state while the guide wire 60 is moved in advance. At this time, a puncture site expanded by pre-dilation is further expanded to a hole diameter through which the catheter 20 can be easily inserted by the tapered part 52 of the flexible part 50. Since the tapered part 52 is made of a flexible material, the puncture site can be reliably expanded without damaging the skin. Moreover, since the dilator 30 is inserted into the catheter 20 as a core material, the catheter assembly 10 is not too soft and is stably inserted to a target position. Furthermore, since the catheter assembly 10 is provided with the physical property inclined part 42 in the dilator 30, the distal end part thereof is not excessively hard, and the risk of blood vessel perforation is reduced. Therefore, the catheter assembly 10 can suppress the blood vessel perforation even when passing through tortuous blood vessel sites in the femoral vein V1, the iliac vein V2, and the vena cava V3. Since the physical property inclined part 42 of the dilator 30 gradually increases in flexibility toward the distal end side, the physical property inclined part exhibits a behavior in which a bending angle increases toward the distal end when a constant force in a direction perpendicular to the axial center of the dilator 30 is applied. As a result, the distal end side of the flexible part 50 and the physical property inclined part 42 can be inserted by exerting a pushing force by the proximal end side of the physical property inclined part 42 and the shaft base part 41 while taking a shape along a relatively tortuous blood vessel shape.

After the distal end of the catheter body 21 reaches the target position of the vena cava V3, the operator stops pushing the catheter assembly 10. Next, the operator rotates the rotation operation unit 34 to release the screwing engagement between the male screw 26 and the female screw and release the connection between the catheter 20 and the dilator 30. Thereafter, the dilator 30 and the guide wire 60 are removed while leaving the catheter 20 in the blood vessel. This enables blood removal and blood feeding via the catheter lumen 24.

As described above, the dilator 30 in the first embodiment is the dilator 30 that can be inserted into the catheter 20, includes the tubular shaft part 40 and the tubular flexible part 50 that is located on the distal end side of the shaft part 40 and is formed of a resin more flexible than the shaft part 40, and includes, in the shaft part 40 and/or the flexible part 50, the physical property inclined part 42 in which the rigidity gradually decreases from the proximal end side toward the distal end side. As a result, since the dilator 30 is harder on the proximal end side than on the distal end side, stable insertability to the blood vessel can be imparted to the catheter 20, and the risk of blood vessel perforation can be reduced since the physical property inclined part 42 is provided. That is, the dilator 30 can achieve both stable insertability of the catheter 20 and a reduction in risk of blood vessel perforation.

Furthermore, in the physical property inclined part 42, the blending of the proximal end side resin A forming a part on the proximal end side with respect to the physical property inclined part 42 and the distal end side resin B forming a part on the distal end side with respect to the physical property inclined part 42 gradually changes from the proximal end side toward the distal end side. As a result, the physical property inclined part 42 in which the rigidity gradually decreases from the proximal end side toward the distal end side can be realized with a stable integral structure.

Furthermore, the flexible part 50 is disposed on the distal end side of a half of the entire length of the dilator 30. As a result, since the proximal end side of the dilator 30 can be suppressed from becoming too flexible, stable insertability into the blood vessel can be effectively imparted to the catheter 20, and the distal end side of the dilator 30 becomes flexible, so that the risk of blood vessel perforation can be effectively reduced.

Furthermore, the flexible part 50 includes, at a distal end part, a tapered part 52 whose outer diameter gradually decreases toward the distal end side. As a result, the distal end part of the flexible part 50 gradually becomes flexible and thin toward the distal end side, so that the risk of blood vessel perforation can be reduced while the insertability of the flexible part 50 is improved.

Furthermore, the physical property inclined part 42 is disposed on the shaft part 40. As a result, since the physical property inclined part 42 is disposed on the proximal end side as compared with the case where the physical property inclined part is disposed in the flexible part 50, a wide range of a distal end region of the dilator 30 is easily bent. Therefore, the risk of blood vessel perforation by the dilator 30 can be reduced.

Furthermore, in a case where the hardness of the proximal end side resin A forming a part on the proximal end side with respect to the physical property inclined part 42 is defined as H_{A} and the hardness of the distal end side resin B forming a part on the distal end side with respect to the physical property inclined part 42 is defined as H_{B}, the hardness ratio is 1.1 ≤ H_{A}/H_{B} ≤ 2.0. As a result, the dilator 30 can achieve both the stable insertability of the catheter 20 into the blood vessel due to the fact that the proximal end side of the shaft part 40 is harder than the distal end side and the reduction in the risk of blood vessel perforation due to the physical property inclined part 42 in a well-balanced manner.

Furthermore, the catheter assembly 10 according to the first embodiment includes: the dilator 30 including the tubular shaft part 40 and the tubular flexible part 50 located on the distal end side of the shaft part 40 and formed of a resin more flexible than the shaft part 40, and including, in the shaft part 40 and/or the flexible part 50, the physical property inclined part 42 in which the rigidity gradually decreases from the proximal end side toward the distal end side; and the catheter 20 connectable to the dilator 30 and covering at least a part of the dilator 30 in a state of being connected to the dilator 30. As a result, since the proximal end side of the catheter assembly 10 is harder than the distal end side, stable insertability to the blood vessel can be imparted to the catheter 20, and the risk of blood vessel perforation can be reduced since the physical property inclined part 42 is provided. That is, the catheter assembly 10 can achieve both stable insertability of the catheter 20 and a reduction in risk of blood vessel perforation.

Furthermore, in a state where the catheter 20 is connected to the dilator 30, the distal end of the physical property inclined part 42 is located closer to the proximal end side than the distal end of the catheter 20. As a result, the physical property inclined part 42 is disposed closer to the proximal end side than the distal end of the catheter 20 in the catheter assembly 10, and the tapered part 52, which is a part where the bending rigidity decreases toward the distal end side, a step part between the distal end of the catheter 20 and the dilator 30, and the physical property inclined part 42 are dispersedly disposed in a wide range of the distal end part of the catheter assembly 10. Therefore, the physical property inclination of the entire catheter assembly 10 can be made gentle, and it is possible to suppress a decrease in the insertability of the catheter 20 into the blood vessel due to a sharp physical property inclination.

### <Second Embodiment>

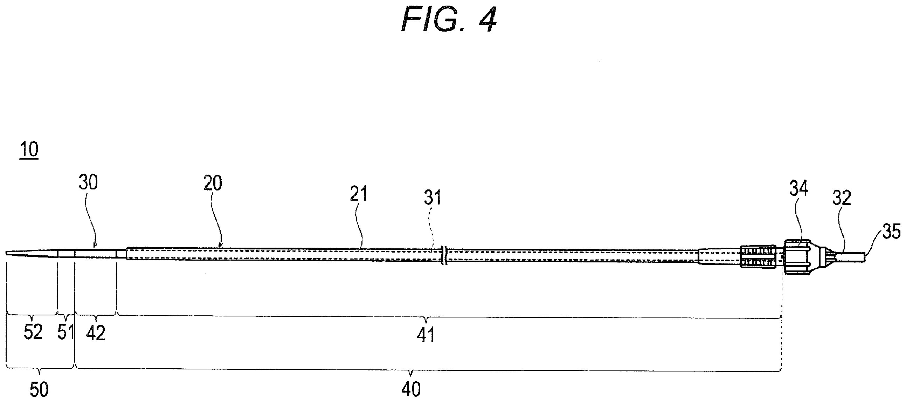

As illustrated in Fig. 4, a catheter assembly 10 according to a second embodiment of the present invention is different from that of the first embodiment only in that a proximal end of a physical property inclined part 42 in an assembled state in which a catheter 20 is connected to a dilator 30 is located closer to a distal end side than a distal end of the catheter 20. That is, the physical property inclined part 42 of the dilator 30 is exposed to the outside without being covered with the catheter 20. As a result, in a case where a catheter body 21 is thin in the radial direction and easily kinks, a wide range (the entire catheter body 21) including the distal end of the catheter 20 can be supported by a part (a shaft base part 41) having higher rigidity on the proximal end side than the physical property inclined part 42 of the dilator 30. Therefore, it is easy to insert the catheter 20 into the blood vessel and indwell the catheter without kinking the catheter.

### [Third Embodiment]

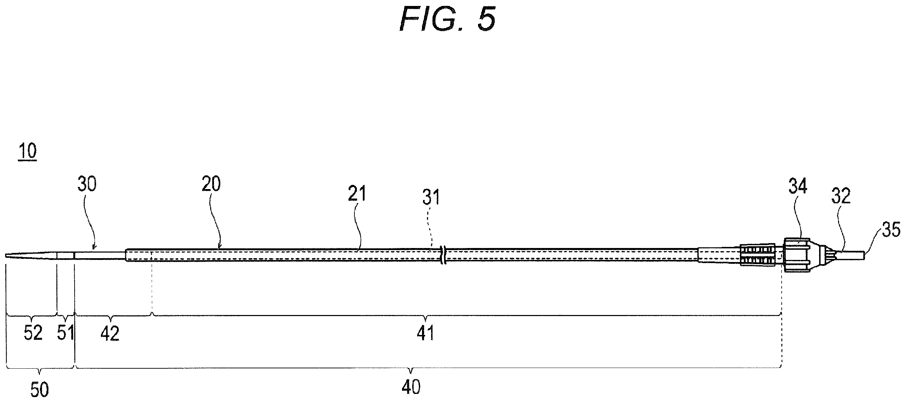

As illustrated in Fig. 5, a catheter assembly 10 according to a third embodiment of the present invention is different from that of the first embodiment only in that a distal end of a catheter 20 is located in the middle of a physical property inclined part 42 in an assembled state in which the catheter 20 is connected to a dilator 30. That is, the distal end of the physical property inclined part 42 of the dilator 30 is located closer to the distal end side than the distal end of the catheter 20, and the proximal end of the physical property inclined part 42 of the dilator 30 is located closer to the proximal end side than the distal end of the catheter 20. Therefore, only the proximal end part of the physical property inclined part 42 of the dilator 30 is covered with the catheter 20, and the distal end part is exposed to the outside without being covered with the catheter 20. As a result, for example, in a case where a material of a catheter body 21 is less than or equal to a hardness of a flexible part 50 of the dilator 30, the catheter 20 is easily bent together with the dilator 30, and a clearance is hardly generated between the catheter 20 and the dilator 30 at the time of puncturing the skin. Therefore, when the catheter assembly 10 is pushed in the distal direction, it is possible to prevent the distal end of the catheter 20 from being caught or turned in the blood vessel.

### [Fourth Embodiment]

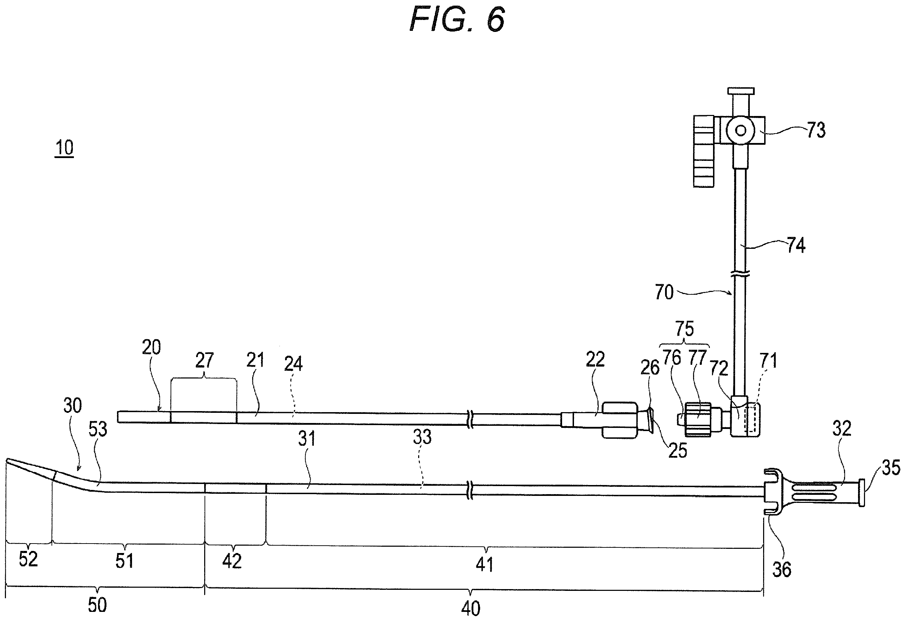

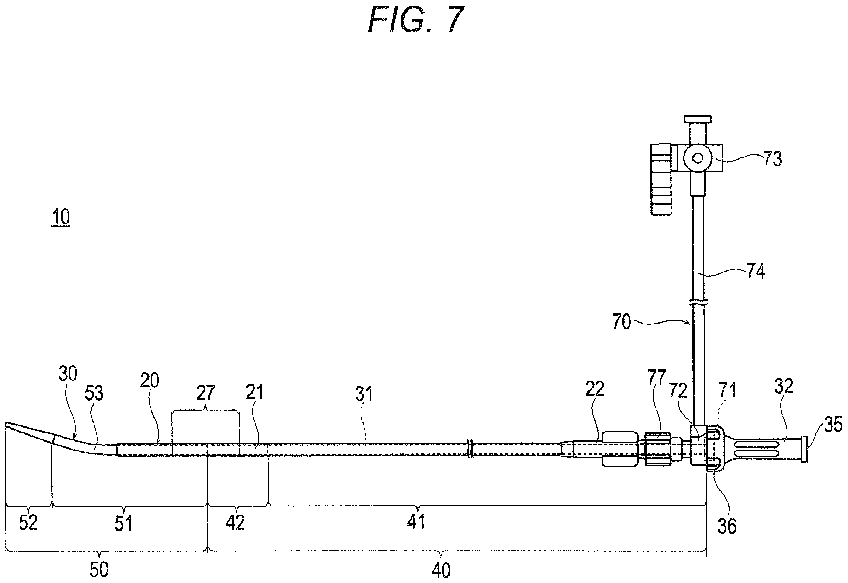

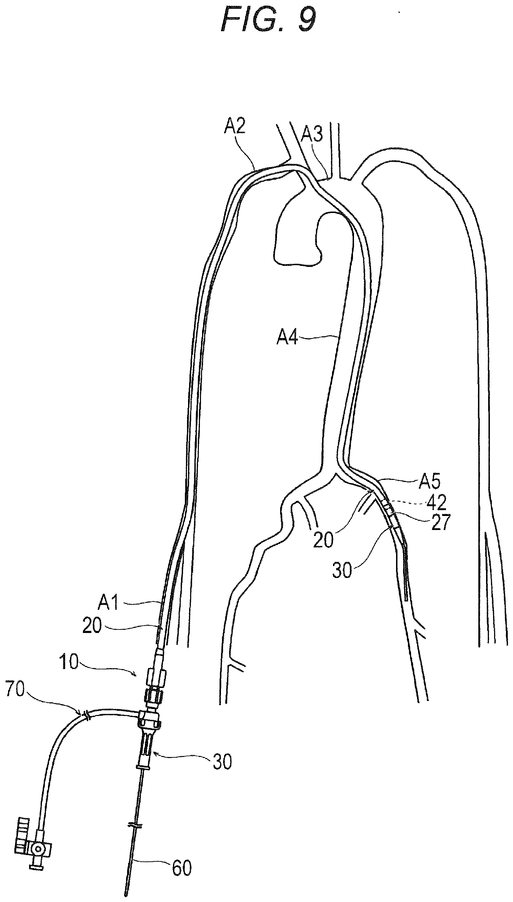

As illustrated in Figs. 6 and 7, a catheter assembly 10 according to a fourth embodiment of the present invention includes, in a catheter 20, a catheter physical property inclined part 27 in which rigidity gradually decreases from a proximal end side toward a distal end side. Note that parts having functions common to the configurations of the first to third embodiments described above are denoted by the same reference signs, and description thereof is omitted. As illustrated in Fig. 9, the catheter assembly 10 according to the fourth embodiment is a product called a "guiding sheath" for inserting from a radial artery A1 to a lower limb artery A5, but the application of the catheter assembly 10 is not limited.

The catheter assembly 10 according to the fourth embodiment includes the catheter 20 that provides a passage for injecting a contrast medium or inserting a medical instrument such as a balloon catheter, a dilator 30, and a hemostasis valve 70. A catheter hub 22 and a dilator hub 32 are not directly connected as in the first to third embodiments, but are indirectly connected via the hemostasis valve 70.

The hemostasis valve 70 includes a valve body 71, a housing 72 accommodating the valve body 71, a three-way stopcock 73 capable of opening and closing and switching a flow path, and a side tube 74 connecting the housing 72 and the three-way stopcock 73. The valve body 71 is a backflow prevention valve for preventing outflow of blood, and a notched hole that can be opened by being deformed is formed in an elastically deformable disk-shaped member. The housing 72 accommodates the valve body 71 on one end side of the through hole, and includes a connector 75 connectable to the catheter hub 22 on the other end side. The connector 75 includes a connecting cylindrical part 76 having an outer peripheral surface that enters a catheter hub opening 25 and can be brought into close contact with an inner peripheral surface of the catheter hub opening 25, and a rotary connector 77 that can rotate an outer periphery of the connecting cylindrical part 76. A female screw that can be screwed with a male screw 26 of the catheter hub 22 is formed on an inner peripheral surface of the rotary connector 77. The side tube 74 connects a lumen on the distal end side of the valve body 71 of the housing 72 and the three-way stopcock 73.

The dilator 30 includes a tubular dilator body 31 and a dilator hub 32 fixed to a proximal end of the dilator body 31. The dilator body 31 includes a tubular shaft part 40 and a flexible part 50 that is more flexible than the shaft part 40 and located on the distal end side of the shaft part 40.

The shaft part 40 includes a shaft base part 41 having uniform bending rigidity along the axial center of the dilator 30, and a physical property inclined part 42 disposed on the distal end side of the shaft base part 41 and having bending rigidity gradually decreasing from the proximal end side toward the distal end side.

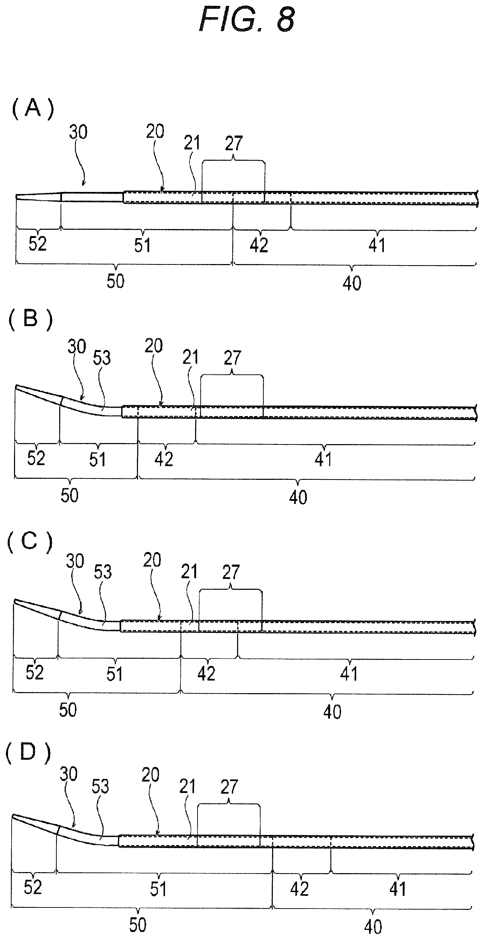

The flexible part 50 is formed with a curved part 53 having a curved axial center. The curved part 53 is effective for changing a direction in which the distal end of the dilator 30 faces by rotating a dilator hub 32 that can be operated by the operator's hand. Note that, as in a first modification example illustrated in Fig. 8(A), the flexible part 50 may not include the curved part 53.

As illustrated in Figs. 6 and 7, the dilator hub 32 includes a plurality of connecting claws 36 that can be connected so as to be hooked on the outer peripheral surface of the housing 72 of the hemostasis valve 70 on the distal end side.

The catheter 20 includes the catheter physical property inclined part 27 in which rigidity gradually decreases from a proximal end side toward a distal end side in a tubular catheter body 21 having flexibility. The catheter body 21 has a three-layer structure including a resin inner layer forming an inner peripheral surface, a resin outer layer forming an outer peripheral surface, and a reinforcing layer disposed between the inner layer and the outer layer. The reinforcing layer is, for example, a coil obtained by spirally winding at least one metal wire or a blade tube obtained by braiding a plurality of metal wires into a tubular shape. A change in rigidity of the catheter physical property inclined part 27 is formed by a change in physical properties of the outer layer. The catheter physical property inclined part 27 can be formed, for example, in the same manner as the physical property inclined part 42 described above. Therefore, for example, in the catheter physical property inclined part 27, the blending of a proximal end side resin C forming a part on the proximal end side with respect to the catheter physical property inclined part 27 and a distal end side resin D forming a part on the distal end side with respect to the catheter physical property inclined part 27 gradually changes from the proximal end side toward the distal end side.

The distal end of the physical property inclined part 42 of the dilator 30 is located closer to the proximal end side than the distal end of the catheter physical property inclined part 27 and is located closer to the distal end side than the proximal end of the catheter physical property inclined part 27. The proximal end of the physical property inclined part 42 of the dilator 30 is located closer to the proximal end side than the proximal end of the catheter physical property inclined part 27. That is, a part of the distal end side of the physical property inclined part 42 of the dilator 30 and a part of the proximal end side of the catheter physical property inclined part 27 overlap in the axial direction of the dilator 30. Therefore, the physical property inclined part 42 of the dilator 30 and the catheter physical property inclined part 27 partially overlap each other without completely matching each other in the axial direction of the dilator 30.

Next, a method of using the catheter assembly 10 according to the fourth embodiment will be described. Here, as illustrated in Fig. 9, a case where the catheter assembly 10 is inserted from the radial artery A1 and indwelled in the lower limb artery A5 will be described as an example.

First, before the catheter assembly 10 is introduced into the blood vessel, as illustrated in Fig. 7, the catheter 20, the hemostasis valve 70, and the dilator 30 are connected to be in an assembled state. When connecting, the connecting cylindrical part 76 of the hemostasis valve 70 is inserted into the catheter hub opening 25, and the rotary connector 77 is rotated to screw the female screw of the rotary connector 77 to the male screw 26 of the catheter hub 22. As a result, the catheter 20 and the hemostasis valve 70 are connected. Next, the dilator 30 is inserted into the housing 72 of the hemostasis valve 70, and the valve body 71 in the housing 72 is inserted into the catheter 20 while being opened. The distal end of the dilator 30 passes through the hemostasis valve 70 and the catheter 20 and protrudes to the distal end side of the catheter 20. Then, the connecting claws 36 of the dilator hub 32 are connected so as to be hooked on the housing 72 of the hemostasis valve 70. As a result, the catheter 20, the hemostasis valve 70, and the dilator 30 are connected. Therefore, when the catheter assembly 10 is inserted into the blood vessel, the catheter 20, the hemostasis valve 70, and the dilator 30 can be integrally operated.

Next, the operator punctures the radial artery A1 by a known method, inserts a short sheath, and performs pre-dilation. Next, a guide wire 60 is inserted into the blood vessel through the short sheath, and the short sheath is removed. Subsequently, the catheter assembly 10 is moved from a subclavian artery A2 to a descending aorta A4 via an aortic arch A3 in the assembled state while the guide wire 60 is moved in advance, and then the catheter assembly is moved to the lower limb artery A5. Therefore, the catheter assembly 10 needs to cross a large tortuosity when reaching the descending aorta A4 from the aortic arch A3. At this time, since the dilator 30 has sufficient rigidity at the proximal end part thereof, the dilator has high torque transmission properties, and the distal end part can be easily oriented. Furthermore, since the dilator 30 is provided with the physical property inclined part 42, the flexibility gradually increases toward the distal end side, so that the distal end part does not become too hard, and the risk of blood vessel perforation is reduced. Furthermore, since the catheter 20 includes the catheter physical property inclined part 27, a distal end part thereof becomes flexible and has high followability to a blood vessel. For this reason, the catheter assembly 10 hardly generates a gap between the dilator 30 and the catheter 20 when crossing a large tortuosity, and can smoothly cross the tortuosity.

Furthermore, in the catheter assembly 10, the dilator 30 is provided with the physical property inclined part 42, the catheter 20 is provided with the catheter physical property inclined part 27, and the positions of the physical property inclined part 42 and the catheter physical property inclined part 27 are shifted in the axial direction. Accordingly, the catheter assembly 10 has sufficient rigidity at the proximal end part as a whole including the catheter 20 and the dilator 30, and the rigidity gradually decreases from the proximal end side toward the distal end side. Therefore, since the catheter assembly 10 has sufficient rigidity at the proximal end part thereof, the catheter assembly has high torque transmission properties and the distal end part can be easily oriented. Moreover, the distal part of the catheter assembly 10 is not too hard, and the risk of blood vessel perforation is reduced.

After the distal end of the catheter body 21 reaches the target position of the lower limb artery A5, the operator stops pushing the catheter assembly 10. Next, the operator disengages the connecting claws 36 of the dilator hub 32 from the housing 72 of the hemostasis valve 70 to release the connection between the hemostasis valve 70 and the dilator 30. Thereafter, the operator leaves the catheter 20 in the blood vessel, and removes the dilator 30 while connecting the hemostasis valve 70 to the catheter 30. When the dilator 30 is removed, the valve body 71 in the housing 72 of the hemostasis valve 70 is closed, and backflow of blood is prevented. This enables a procedure for diagnosis and treatment by inserting a medical instrument corresponding to a treatment site using the catheter 20 and the hemostasis valve 70.

As described above, the catheter 20 includes the catheter physical property inclined part 27 whose rigidity gradually decreases from the proximal end side toward the distal end side. As a result, the distal end part of the catheter 20 becomes flexible and has high followability to the blood vessel. For this reason, the catheter assembly 10 hardly generates a gap between the dilator 30 and the catheter 20 when crossing a large tortuosity, and can smoothly cross the tortuosity.

Furthermore, in a state where the catheter 20 is directly or indirectly connected to the dilator 30, the distal end of the physical property inclined part 42 of the dilator 30 is located closer to the proximal end side than the distal end of the catheter physical property inclined part 27, and the proximal end of the physical property inclined part 42 of the dilator 30 is located closed to the proximal end side than the proximal end of the catheter physical property inclined part 27. As a result, the catheter assembly 10 includes a part where rigidity gradually decreases from the proximal end side toward the distal end side close to both the catheter 20 and the dilator 30. For this reason, rigidity of the catheter assembly 10 gradually decreases from the proximal end side toward the distal end side as a whole including the catheter 20 and the dilator 30. Therefore, the catheter assembly 10 can effectively reduce the risk of blood vessel perforation while having high torque transmission.

Note that, as a second modification example of the fourth embodiment, as illustrated in Fig. 8(B), the distal end and the proximal end of the physical property inclined part 42 of the dilator 30 may be located closer to the distal end side than the distal end of the catheter physical property inclined part 27. Furthermore, as a third modification example of the fourth embodiment, as illustrated in Fig. 8(C), the distal end of the physical property inclined part 42 of the dilator 30 may be located closer to the distal end side of the distal end of the catheter physical property inclined part 27, and the proximal end of the physical property inclined part 42 of the dilator 30 may be located closer to the proximal end side than the distal end of the catheter physical property inclined part 27 and to the distal end side than the proximal end of the catheter physical property inclined part 27. Furthermore, as a fourth modification example of the fourth embodiment, as illustrated in Fig. 8(D), the distal end and the proximal end of the physical property inclined part 42 of the dilator 30 may be located closer to the proximal end side than the proximal end of the catheter physical property inclined part 27. Furthermore, in the second to fourth modification examples of the fourth embodiment, the dilator 30 may not include the curved part 53 as in the first modification example illustrated in Fig. 8(A).

Note that, in the fourth embodiment, an aspect in which the hemostasis valve 70 separated from the catheter 20 is used has been described, but an aspect in which a hemostatic valve body is provided in the hub 22 of the catheter 20, and the dilator 30 and the hub 22 can be directly connected may be adopted.

Note that the present invention is not limited to the embodiment described above, and various modifications may be made by those skilled in the art within the technical idea of the present invention. For example, at least one of the catheter body 21 and the dilator body 31 may be configured to be curved. Furthermore, the catheter assembly 10 is not limited to the ECMO, and may be applied to, for example, an introducer, a guiding sheath, a movable introducer provided with a mechanism capable of changing a direction of the distal end part by operating the proximal end part, or the like. In these applications, the outer diameter, length, hardness, and the like of the catheter or dilator of the catheter assembly can be changed according to each application.

Note that the present application is based on Japanese Patent Application No. 2021-102182 filed on June 21, 2021, the disclosure content of which is incorporated herein by reference in its entirety.

### Reference Signs List

- 10: Catheter assembly
- 20: Catheter
- 27: Catheter physical property inclined part
- 30: Dilator
- 40: Shaft part
- 42: Physical property inclined part
- 50: Flexible part
- 52: Tapered part
- H_{A}: Hardness of proximal end side resin
- H_{B}: Hardness of distal end side resin

## Claims

1. A dilator insertable into a catheter, the dilator including a tubular shaft part, and a tubular flexible part located on a distal end side of the shaft part and formed of a resin more flexible than the shaft part, and
comprising, in the shaft part and/or the flexible part, a physical property inclined part in which rigidity gradually decreases from a proximal end side toward a distal end side.

2. The dilator according to claim 1, wherein in the physical property inclined part, blending of a resin forming a part on a proximal end side with respect to the physical property inclined part and a resin forming a part on a distal end side with respect to the physical property inclined part gradually changes from the proximal end side toward the distal end side.

3. The dilator according to claim 1 or 2, wherein the flexible part is disposed on a distal end side of a half of a total length of the dilator.

4. The dilator according to any one of claims 1 to 3, wherein the flexible part includes, at a distal end part, a tapered part in which an outer diameter gradually decreases toward a distal end side.

5. The dilator according to any one of claims 1 to 4, wherein the physical property inclined part is disposed in the shaft part.

6. The dilator according to claim 2, wherein a hardness ratio is 1.1 ≤ H_{A}/H_{B} ≤ 2.0, where H_{A} is defined as a hardness of the resin forming the part on the proximal end side with respect to the physical property inclined part, and H_{B} is defined as a hardness of the resin forming the part on the distal end side with respect to the physical property inclined part.

7. A catheter assembly comprising:
a dilator including a tubular shaft part, and a tubular flexible part located on a distal end side of the shaft part and formed of a resin more flexible than the shaft part, and including, in the shaft part and/or the flexible part, a physical property inclined part in which rigidity gradually decreases from a proximal end side toward a distal end side; and
a catheter connectable to the dilator and covering at least a part of the dilator in a state of being connected to the dilator.

8. The catheter assembly according to claim 7, wherein a distal end of the physical property inclined part is located closer to a proximal end side than a distal end of the catheter in a state where the catheter is connected to the dilator.

9. The catheter assembly according to claim 7, wherein a proximal end of the physical property inclined part is located closer to a distal end side than a distal end of the catheter in a state where the catheter is connected to the dilator.

10. The catheter assembly according to claim 7, wherein a distal end of the catheter is located in a middle of the physical property inclined part in an axial direction of the dilator in a state where the catheter is connected to the dilator.

11. The catheter assembly according to claim 7, wherein the catheter includes a catheter physical property inclined part in which rigidity gradually decreases from a proximal end side toward a distal end side.

12. The catheter assembly according to claim 11, wherein in a state where the catheter is directly or indirectly connected to the dilator, a distal end of the physical property inclined part of the dilator is located closer to a proximal end side than a distal end of the catheter physical property inclined part, and a proximal end of the physical property inclined part of the dilator is located closer to a proximal end side than a proximal end of the catheter physical property inclined part.
